# EUROPEAN PATENT APPLICATION

(11) **EP 3 093 054 A1**
(43) Date of publication of application: **16.11.2016**
(21) Application number: 15167208.6
(22) Date of filing: 11.05.2015
(51) Int. Cl.: B01D 25/26

(54) **FLUID TREATMENT MODULE AND ASSEMBLY**

(71) Applicant: Pall Corporation, Port Washington, NY 11050 (US)
(72) Inventor: Friedrich, Esther, 55545 Bad Kreuznach (DE); Meng, Manuela, 55776 Ruschberg (DE); Leibnitz, Rüdiger, 55543 Bad Kreuznach (DE)
(74) Representative: Hoeger, Stellrecht & Partner Patentanwälte mbB

(57) **Abstract**

A fluid treatment module (200) is proposed comprising a fluid treatment element (202), a fluid inlet for non-treated fluid and a fluid outlet for treated fluid,
said fluid treatment element comprising a body of a fluid pervious treatment medium with a first and a second face positioned at opposite sides of the body. The body comprises a plurality of channels extending in a direction from the first face to the second face.

A number of channels of a first type of said channels is open at one end located at said first face of the body and closed at the other end located adjacent to second face.

A number of channels of a second type of said channels is open at one end located at said second face of the body, and closed at the other end located adjacent to said first face.

The channels of the first type are separated from the channels of the second type by portions of the treatment medium,
one of said types of channels receiving non-treated fluid and communicating with the fluid inlet, the other one of said types of channels collecting treated fluid and communicating with said fluid outlet.

## Description

The present invention relates to a fluid treatment module and a fluid treatment assembly incorporating same as well as a process for treating a fluid making use of such a modules and assembly.

### BACKGROUND OF THE INVENTION

Generally, fluids with a high load of contaminants, e.g., particulate matter, colloids, macro-molecules, biological cells, cell debris, abrasion particles and the like, present considerable challenges for fluid treatment modules and fluid treatment assemblies, especially when large quantities of fluids are to be treated and/or high quality standards are to be met. Such challenges especially exist when fluids in the pharmaceutical industry, in the field of food and beverage etc. are to be treated. Especially the treatment of reaction media of the biotech industry are to be mentioned in this context.

The biotech industry has been rapidly changing over the past 10 years. Cell counts in biotech fluids, i.e., reaction media are getting higher (e.g., an increase of about 25 Mio cells per ml); cells (e.g., Chinese hamster ovary (CHO) cells) are getting larger (e.g., between 15 and 27 µm); the PCV (packed cell volume) is getting higher, (e.g., about 10%); and early removal of host cell proteins (HCP) and DNA from the reaction media is becoming more important.

In order to cope with the above mentioned challenges, recently single use fluid treatment capsules have been used for clarification of the reaction media, but the considerable number of filter modules needed when conventional fluid treatment capsules are used increases costs, footprint, the need for rinse water, and the potential of product losses.

Centrifugation is another technique conventionally used to clarify biotech fluids, but it does not fit well with the single use requirements and the resulting shear forces during centrifugation could disrupt cells and increase HCP and DNA content in the fluids.

Depth filtration, alone, can be effective, but it typically requires too much filter surface area and can increase treatment/filtration time. Other techniques, such as the use of flocculation in combination with depth filtration, are also effective, but the addition of chemicals (e.g., flocculating agents) can be problematic in biotech processes.

The object of the present invention is to provide reliable means to treat, especially clarify biotech fluids in a cost effective manner.

### SUMMARY OF THE INVENTION

According to a first aspect the present invention relates to a fluid treatment module comprising a fluid treatment element, a fluid inlet for non-treated fluid and a fluid outlet for treated fluid, said fluid treatment element comprising a body of a fluid pervious treatment medium with a first and a second face said first and second face being positioned at opposite sides of the body, said body comprising a plurality of channels extending in a direction from the first face to the second face, wherein a number of channels of a first type of said plurality of channels is open at one end located at said first face of the body and closed at the other end located adjacent to second face, wherein a number of channels of a second type of said plurality of channels is open at one end located at said second face of the body, and closed at the other end located adjacent to said first face, wherein said channels of the first type are separated from the channels of the second type by portions of the fluid pervious treatment medium, one of said types of channels being feed channels communicating with the fluid inlet of said fluid treatment module, the other one of said types of channels being outlet channels communicating with said fluid outlet of said fluid treatment module.

The fluid treatment module according to the present invention provides means to effectively clarify the biotech fluids, e.g., reaction media, by removing unwanted components. The fluid treatment modules can be produced in a cost effective manner, provide a substantially increased capacity, and allow the treatment of the fluid to be treated, especially clarified to be completed in a reasonable time.

Generally any fluid with a high load of contaminants or unwanted components can advantageously be treated and thereby clarified by the inventive treatment modules and assemblies. This holds true not only for fluids of the pharmaceutical especially the biopharmaceutical field but also for fluids of the fields of food and beverage.

Advantageously, the fluid treatment module of the present invention, although it may be produced cost-effective, provides for high-throughput (e.g., liters per module), reduces foot-print of a system incorporating the module, requires less rinsing media, reduces waste material, may be provided with low contents of extractables, and has a potential for less product loss.

As compared to the use of centrifuges, the present invention offers additional advantages, e.g., it is ready to use, provides for improved recovery and higher capacity, better protection of downstream sterile filters, especially sterile membrane filters, simple process, lower cost, and is suitable for single use, thereby avoiding cross contamination, having no need for cleaning validation, requires less maintenance efforts, provides better and easier upscaling, has a higher flexibility, etc.

Treatment systems incorporating the inventive fluid treatment modules and assemblies allow for an efficient use of the modules and provide the possibility of treating large amounts of fluids in single use applications.

The fluid treatment modules can be used to treat a fluid in a variety of treatment techniques, such as, depth filtration, adsorptive separation, chromatographic separations, surface (sieving) filtration, or any combinations thereof.

### DETAILED DESCRIPTION OF THE INVENTION

The inventive fluid treatment module may be provided with a body of treatment medium of a unitary structure. This aspect is of interest especially when a smaller quantity of fluid is to be treated at a time. Assembly is facilitated by the use of unitarily structured bodies of the fluid treatment module. The first and second faces of the body of treatment medium are preferably of a flat configuration and arranged in parallel to one another.

In another preferred embodiment of the present invention said body of treatment material may comprise a plurality of flat layers of a sheet material, said layers comprising openings forming said plurality of channels once the layers are stacked.

This type of treatment module may easily be adapted in its treatment capacity to a broad range of fluid volumes, merely by increasing the number of stacked layers of sheet material forming said body.

The fluid treatment element of the fluid treatment module of the present invention may comprise one or more layers of a first type of layers located at and positioned in parallel to the first face of the body, said first type of layers having openings corresponding only to one type of channels and/or said fluid treatment element comprises one or more layers of a second type of layers located at and positioned in parallel to the second face of the body, said second type of layers having openings corresponding only to the other type of channels.

While the various layers may be made of the same type of material, the present invention provide the possibility to use layers made of different types of material which further increases the flexibility to adapt the fluid treatment modules to specific needs in a given application.

According to this aspect of the invention the first and second type of layers may accommodate in between them a stack of a broad range of different numbers of identically formed layers of sheet material in order to cope with the various demands.

More preferably, said first and second types of layers are made of a fluid treatment material the composition and/or structure of which is preferably similar to or identical with the treatment medium of the body of the treatment element. Thus a rather homogeneous body of treatment material may be provided facilitating the manufacture and especially the quality control and validation of the module.

According to a further aspect of the present invention the cross sections of each type of channels within the body of treatment material are substantially of the same size. Manufacturing equipment may be provided at relatively low costs.

Alternatively, the inventive fluid treatment module may have cross sections of the channels communicating with the fluid inlet that are of a larger size than the cross sections of the channels communicating with the fluid outlet. Such a feature takes into account that the capacity of the module may be significantly increased, since the module can more easily cope with deposits occurring during the treatment of a fluid within the body of treatment material.

More preferably, the difference in size of the cross sections amounts to about 10 % or more, based on the size of the cross section of the channels communicating with the fluid outlet.

According to a still further aspect of the present invention the number of channels communicating with the fluid inlet may be higher than the number channels communicating with the fluid outlet. This may alternatively or additionally provide an increase in capacity of the treatment module.

More preferably, the number of channels communicating with the fluid inlet is about twofold or more as compared to the number of channels communicating with the fluid outlet.

In the alternative the number of channels communicating with the fluid inlet may be lower than the number of channels communicating with the fluid outlet. Especially in such a configuration it is preferred when the cross section of the channels communicating with the fluid inlet is significantly larger, e.g., about 10% or more, preferably about 20% or more, than the cross section of the channels communicating with the fluid outlet.

The arrangement of the two types of channels within the body of fluid pervious treatment material can be selected from a variety of patterns.

An alternating arrangement of the two types of channels in a plurality of concentric circles has provided good results. In case of treatment modules incorporating a plurality of layers correct assembly of the layers to form the body of treatment material is greatly facilitated.

In an alternative preferred arrangement the channels are provided in the fluid treatment material in separate parallel rows for each type of channels. Creating the openings in the plurality of layers providing the channel structure in the body of treatment material is greatly facilitated and may be achieved in cost effective manner. This also provides an easy means to ensure that the different types of channels are arranged in regular, closely controlled distances from one another which on the other hand improves the accuracy and reliability of the treatment results.

More preferably, the channels communicating with the fluid inlet are arranged in groups of two or more adjacent rows, whereas the channels communicating with the fluid outlet are arranged in groups of a lesser number of rows. Thus more than one row of channels receives the fluid to be treated which may be used to reduce fluid flow on the upstream side of the treatment material which may in specific applications provide for better treatment results.

In the alternative the number of rows of the channels communicating with the fluid inlet is lesser than the number of rows of channels communicating with the fluid outlet.

The fluid treatment module of the present invention may be designed as a filter module. The fluid treatment material may then be selected from a depth filter material. Preferably, the body of fluid treatment material composed of a depth filter material is maintained in a compressed state. This is especially preferred when the body of treatment material is composed of a plurality of stacked depth filter layers. It has been found out that by compression of the plurality of layers of depth filter material it is easily possible to avoid bypasses within the body and thereby provide high quality results in the treatment of the fluid.

More preferably, the compression of the fluid treatment material is such that the thickness of the body of treatment material amounts to about 90 % or less of the thickness of the non-compressed depth filter material.

The fluid treatment module comprises according to a further aspect of the present invention a fluid treatment material comprising a matrix including a compressible material and/or a material which swells in contact with the fluid to be treated.

Another option to prevent bypasses resides in positioning sealing strips on the surface of the layers of fluid treatment material in between the areas accommodating the first and second type of channels. The compression of the fluid treatment material can then be reduced to a low level or even completely avoided.

According to a further alternative, the surfaces of the two adjacent layers may be glued together thereby providing a sealing effect to fluidly separate the two types of channels in a way which also may need no compression of the fluid treatment material.

Still in a further alternative, a sealing layer may be positioned in between two adjacent layers of fluid treatment material to keep the two types of channels fluidly separate. Basically, any material which can work as a seal/gasket can be used, e.g., silicone material.

In a further preferred embodiment of the present invention the fluid treatment module comprises a fluid treatment material comprising a matrix incorporating an additive, said additive being preferably in particulate form, preferably wherein the additive is present in an amount of about 15 to about 70 % by weight based on the weight of the fluid treatment material. The properties and treatment results may be adjusted to a specific application for which a treatment module is designed by use of additives incorporated in a matrix of the treatment material.

Preferably, the additive of the fluid treatment material is selected from a porous particulate additive, a filter aid, and/or a treatment agent.

Furthermore the additive preferably comprises a reactive agent, an adsorptive agent.

More specifically, the additive may preferably be selected from kieselguhr, perlite, fine cellulose, micro-crystalline cellulose, nanofibers, activated carbon, adsorber beads, fibrets and fibrids.

If very large particles are to be removed from the feed, a cellulosic matrix alone not including additives can be used as well.

In a number of cases it is preferred that the fluid treatment material of the body of the fluid treatment module comprises an organic polymer material which forms a part of the matrix of the treatment material or medium.

For a large number of applications it is preferred that the fluid treatment material is selected from sterilizable material.

According to a further aspect of the present invention the fluid treatment module comprises two end pieces to be positioned in sealing contact with said first and second faces, said end pieces preferably providing the fluid inlet and fluid outlet of the module, respectively.

One embodiment of the present invention relates to a fluid treatment module including a feed distribution device including a feed plate having a plurality of feed openings, the feed plate being positioned such that the plurality of feed openings fluidly communicate with one type of channels of the body of treatment material.

In another embodiment of the present invention, the fluid treatment module includes a fluid collection device including a fluid collection plate having a fluid discharge opening and a plurality of fluid openings providing fluid communication between one type of channels of the body of the fluid treatment element and the fluid outlet, e.g., filtrate discharge outlet, of the module.

According to another aspect of the inventive concept the fluid treatment module may be provided with a capsule accommodating the fluid treatment module.

According to a further aspect of the present invention, the module may comprise clamping means positioned on the outer peripheral surface of the module, said clamping means preferably maintaining the fluid treatment material in a compressed state.

According to another aspect of the present invention, a fluid treatment assembly is provided which comprises two or more fluid treatment modules of the present invention, the modules being arranged in a stack. Thereby again it is easy to adapt the treatment capacity of an assembly to the volume and/or nature of the fluid to be treated and the challenges resulting therefrom.

Preferably, each of the modules of the assembly comprise a central duct which, when the modules are stacked, form a common central conduit of the assembly which may be used to provide all stacked modules in a simple and efficient manner with the fluid to be treated. Alternatively, the common central conduit may be designed to receive treated fluid from all of the stacked modules.

In yet another embodiment, the fluid treatment assembly includes a retention device for retaining and/or compressing the fluid treatment material of the fluid treatment modules, e.g., compressing the layers or sheets of a depth filter material. Such a retention device may substitute individual clamping means of the individual modules.

A still further aspect of the present invention resides in a process for treating a fluid. The process comprises arranging a fluid treatment module or a fluid treatment assembly of the present invention such that the fluid inlet(s) of the module(s) is/are arranged on top and the channels of the module(s) are oriented in an about vertical direction and feeding the fluid to be treated into the feed inlet of the module and the assembly, respectively.

Preferably, the treatment process comprises capturing sediment from the non-treated fluid in the channels which are in fluid communication with the fluid inlet.

### The Fluid Treatment Module

The fluid treatment module according to the present invention preferably includes a feed distribution device including a feed plate for feed supply and feed distribution for non-treated fluid, and/or a collection device for receiving treated fluid, e.g., filtrate, including a fluid collection plate for collection of treated fluid and conducting the treated fluid to a common outlet, e.g., to a central duct of the module.

Preferably, a plurality of plate shaped fluid treatment material, e.g., sheets, is arranged between the plates. The sheets are preferably comprising openings or holes, which were created by perforation.

The sheets are preferably arranged in such a manner that the holes of stacked layers form continuous channels within the body of stacked sheets. One type of channels distributes the feed within the body of the fluid treatment element and the other type of ducts collects the treated fluid, e.g., filtrate, within the body of the fluid treatment element. The feed channels are providing an open end towards the feed distribution plate and are sealed against the filtrate plate. The filtrate channels are provided with open ends towards the filtrate collection plate and are sealed against the feed plate. The only way for the fluid to get from the feed to the filtrate ducts is to pass through the fluid treatment medium.

### The Fluid Treatment Element

The fluid treatment element often includes two or more layers or sheets of fluid pervious treatment medium, also called material. The fluid treatment medium or material can be, e.g., a depth filter material optionally comprising various components, e.g., a mixture of diatomaceous earth (DE), perlite, cellulose, binder, etc., and can be manufactured from, e.g., a fibrous material, especially woven or non-woven materials, e.g., melt-blown non-woven materials.

One particular example of the fluid treatment material includes a cellulose fiber based matrix and a filtration active substance (e.g. kieselguhr, perlite, fine fibers), which is immobilized within the cellulose fiber matrix.

Depth filter material used as fluid treatment material is preferably stabilized in its structure, especially its micro-structure, by adding a binder material. Suitable binder materials include, e.g., polyamido amine epichlorohydrine, polyethylene aimine, polyvinyl amine, polyisocyanat and melamin formaldehyd. The use of polyamido amine epichlorohydrine, polyethylene imine and polyvinyl amine is especially preferred for food and beverage applications.

For some applications it may be beneficial to modify the sheets of treatment material in order to get a higher stability. The addition of a small amount of special fibers (e.g., about 1 to about 30% by weight, preferably about 3 to about 20% by weight of polyethylene (PE) containing or based fibers) may be advantageous.

A smooth surface of the layers of fluid treatment material is generally advantageous in order to provide for sufficient sealing between adjacent sheets.

Preferably, the fluid treatment material shows some degree of swelling due to, e.g., the swelling of the fibers incorporated in the treatment material, when in contact with the fluid to be treated which facilitates a sufficient sealing between the layers. The mechanical properties of the sheet material, e.g., tensile strength, resistance against compression may be advantageously improved by adding a certain amount of PE fibers to the treatment material, which may be molten together.

According to one concept of the present invention each sheet includes two kinds of openings, one kind serving as feed openings, the other serving as fluid receiving openings, e.g., filtrate openings. The sheets can be arranged (e.g., in stacked configuration) such that the openings of each type of each sheet are aligned to form the feed channels and treated fluid receiving channels, respectively. Both types of channels are to be closed on one end and open at the other end. This may be implemented in different ways.

One option may be to cover the upstream and downstream faces of the body of fluid pervious treatment medium with a thin fluid impervious layer, e.g., a film, one of said fluid impervious layers comprising openings matching with the cross-sections of the channels of one type of channels, e.g., non-treated fluid receiving channels, and the other fluid impervious layer comprising openings matching with the cross-sections of the channels of the other type of channels, e.g., treated fluid receiving channels.

Alternatively, a sheet or sheets of a fluid pervious medium may on one end face of the body contain feed openings only communicating with the non-treated fluid receiving channels, and a sheet or sheets on the other end face of the body include treated fluid draining openings only communicating with the treated fluid receiving channels.

Typically, about 5 to about 40 sheets of fluid treatment material may be used in a multiple layer structured body.

The diameter of the fluid treatment element when provided in a cylindrical form may range from, e.g., about 65 mm for small scale applications, to about 400 mm for large scale applications.

The number of sheets or layers used is just limited by the weight of the module, especially in the wet state, because easy handling of the modules is of quite some import, and, in case the fluid treatment medium layers need to be compressed to avoid bypasses, the number is limited by the required clamping force to be applied for compression.

The distance in between the feed channels and the channels receiving treated fluid can be selected from a broad range of distances. The distance measured at the circumferences of the channels preferably is about 2 mm or more. Thus, a stay in between the two types of channels provides for a sufficient path length for the fluid to be treated when the same migrates through the bulk of the treatment medium such that a sufficient treatment effect can be provided. For example, in one embodiment, the distance may be about 4 mm.

The openings in the layers can be created by punching/perforation (for lower cost and more open surfaces at the cylindrical walls created by the punching/perforation step), e.g., in one embodiment, the perforations of the sheets can be produced by punching every row of openings individually, alternatively punching of all of the openings of a sheet is done simultaneously.

The design of an inventive fluid treatment assembly preferably comprises in addition to a stack of fluid treatment modules a clamping means for compressing the sheet material of the stacks of the modules.

The fluid treatment, e.g., filtration, occurs preferably by inducing an edge flow of the fluid to be treated through the bulk of the treatment medium sheets, i.e., by directing the fluid flow from the feed holes/channels through the bulk of the fluid treatment medium to the holes/channels receiving the treated fluid (e.g., filtrate).

The plurality of openings in the sheets of fluid treatment material and subsequently the channels formed by the openings in a stacked assembly of the sheets can have cross sections selected from a broad variety of geometries.

The openings forming the channels of both types may have polygonal, especially rectangular, circular or oval cross sections. While the openings could be provided, e.g., in the form of elongated slots, considering the mechanical stability of the body of treatment medium under various conditions during operation of the fluid treatment module, a multiple openings, e.g., circular openings, of a limited cross section separated by narrow stays (e.g., about 1 mm) instead of an elongated slot are preferable.

For example, the feed openings constituting the feed channels may have a larger diameter, preferably in the range of from about 2 mm to about 10 mm, e.g., about 5 mm in diameter, than the fluid openings constituting the treated fluid receiving channels which preferably have a diameter of from about 1.5 mm to about 8 mm, e.g., about 2 mm in diameter. The distance of the two adjacent openings of the same type of channels may be, e.g., about 1 mm or more. The stays thereby provided in between adjacent openings of the same type serve to stabilize the structure of the body of treatment medium. In principle, the stays are not needed to provide the basic treatment/filtration functionality. Due to stability considerations the use of stays is preferred.

The openings can be arranged in a variety of configurations such as, e.g., the openings can be arranged in rows, especially linear rows, or in concentric rings each row or circle comprising one type of openings only.

In still another preferred arrangement, the openings of the one type of channels may be surrounded by a multiplicity of openings of the other type of channels which may also be provided in a continuous pattern.

All configurations will work well concerning fluid treatment performance.

However, the configuration in linear rows is preferred due to cost effectiveness and manufacturing feasibility considerations.

When using a plurality of layers of fluid treatment medium it is preferred that the layers or sheets are held in position during all the manufacturing steps and permanently compressed in the final module in order to prevent a bypass.

Compression of the sheet material, especially when it is in the form of a depth filter material, by about 20% in the dry state is recommended to ensure a sufficient sealing effect in the wet state. Thereby, still a sufficient compression in the wet state may be provided, since many of the preferred treatment materials get softer upon contact with the fluid to be treated.

However, the percentage of compression must be balanced. A too high compression could result in a reduced service life due to premature blockage, whereas too low compression could result in bypasses between adjacent sheets, resulting in a turbidity breakthrough. Furthermore, it is preferred that the compression is substantially uniformly distributed over the whole of the fluid treatment element.

### The Feed Distribution Plate

When a feed distribution plate is used it must be arranged such that feed fluid can enter the open ends of the feed channels of the fluid treatment element, and the channels collecting the treated fluid at the same time must be securely sealed off at their ends adjacent to the feed distribution plate. This effect may in some embodiments be accomplished by the feed distribution plate.

A permanent compression of the stack can be provided by the feed distribution plates (together with, e.g., clamping or compression means).

In an alternative embodiment, the feed distribution plate regularly distributes fluid to be treated to the upstream surface of the fluid treatment element which comprises one or more upstream layers of fluid pervious treatment medium provided only with openings communicating with the feed channels. The one or more sheets of fluid pervious treatment medium then cover or close the ends of the channels receiving treated fluid.

The feed distribution plate has to be as thin as possible in order to minimize costs and to maximize the active filter area provided by a certain volume of a fluid treatment module.

### The Fluid Collection Plate

In case a fluid collection plate is used it must be arranged such that the treated fluid, e.g., filtrate, from the channels collecting the treated fluid can enter the plate and be directed to, e.g., a central duct of the fluid treatment element. The feed channels may be at one end sealed off by the fluid collection plate.

Alternatively, the fluid collection plate may support one or more layers of fluid pervious treatment medium at the downstream side of the fluid treatment element, said layers being provided only with openings communicating with the channels collecting the treated fluid. The channels receiving the non-treated fluid may then be covered or closed by these layers.

The filtrate collection plate may cooperate with the feed distribution plate to provide a permanent compression of the stack (together with, e.g., clamping or compression means).

As has been noted for the feed plate, the filtrate collection plate must be as thin as possible in order to minimize costs and to maximize the active filter area provided by a certain volume of a fluid treatment module.

Generally, any plastic material (preferably, glass fiber filled polypropylene (PP)) having sufficient mechanical strength and stiffness in a dry and wet state is suitable for the manufacture of the feed distribution and fluid collection plates. Other aspects derived from regulations concerning, e.g., the selection of materials to come in contact with food or biotech fluids (e.g., Plastic Class VI) may be considered.

For example, the deflection of the feed and fluid collection plates under load must be small in order to ensure an even compression for the whole module.

Preferable polymer materials for manufacturing the feed and fluid collection plates are selected from polypropylene (PP) and polycarbonate (PC) materials.

Preferred additives to improve the mechanical properties of the polymer materials include glass fibers and talcum.

While polypropylene materials often will require the use of reinforcing additives, polycarbonate materials may be used in a large number of cases without such reinforcing additives.

A specific example of a suitable reinforced polymer material is glass-fiber reinforced PP having a glass fiber content of about 30% by weight or more, e.g., about 40 % by weight.

Additional considerations for selecting a suitable material for manufacturing the feed distribution an fluid collection plates include:
a) the materials must meet the biological safety tests (e.g., Plastic Class VI, cytotoxicity test, haemolysis test);
b) the extractables must be below certain limits;
c) the module must be stable against autoclaving, steaming and/or hot water sanitization;
d) the module must be stable against gamma sterilization.

Other materials, e.g., other plastics, stainless steel, etc. can also be used, although for single use options, it is preferable to use a polymeric material.

### The Clamping Means

According to one embodiment, the clamping means includes a plurality, e.g., 4 to 12 clamps regularly arranged around the circumference of the module to permanently retain (and optionally) compress the fluid treatment element at its outer circumference.

Adequate compression may be obtained by the stiffness of the plates, and the compression at the inner core, e.g., at the central duct, of the module.

Sufficient compression may be provided by other means. For example, the module may be incorporated in a housing or capsule which may provide the necessary compression forces. In other cases a spindle may be used to apply a sufficiently high compressing force to a plurality of layers forming the body of treatment medium.

Alternatively or additionally, a retention device may be used to hold together two or more modules in a fluid treatment assembly. In such an embodiment sometimes separate clamping means at the individual modules may no longer be needed.

### BRIEF DESCRIPTION OF THE DRAWINGS

- Figure 1: shows a schematic representation of a first embodiment of a fluid treatment element of an inventive treatment module;
- Figure 2: shows a schematic representation of a second embodiment of a fluid treatment element of an inventive treatment module in an exploded view;
- Figure 3: shows a schematic representation of an inventive assembly comprising two modules of the present invention in an exploded view;
- Figures 4 to 7: show a specific embodiment of an inventive fluid treatment module and its various components in different views; and
- Figures 8 and: 9 show a further specific embodiment of an inventive fluid treatment module and its components in different views.

### DETAILED DESCRIPTION OF THE DRAWINGS

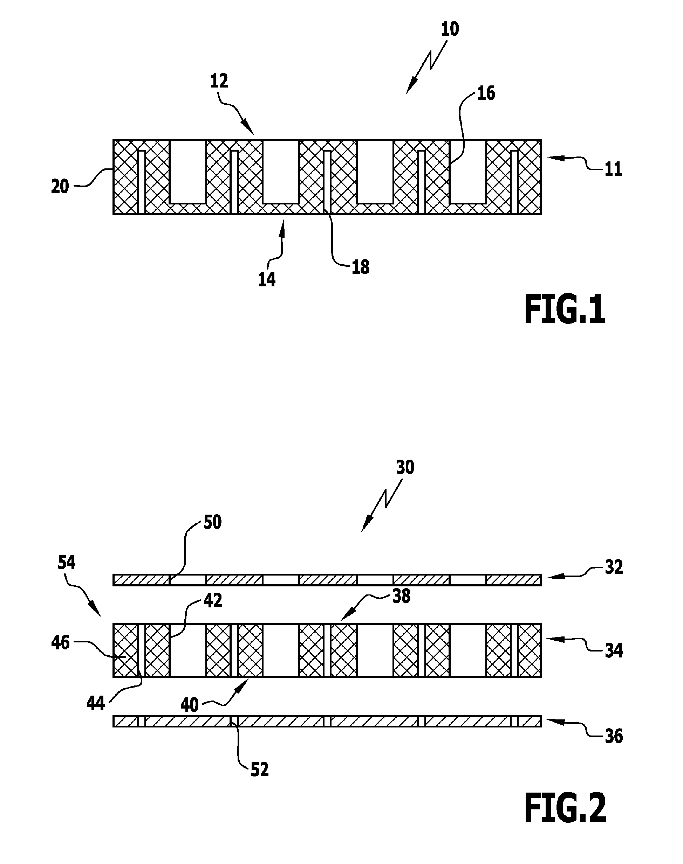

Figure 1 shows a first embodiment of a fluid treatment element 10 designed for an inventive fluid treatment module.

The element 10 is of a substantially flat or plate shaped structure and comprises a body 11 of a fluid pervious treatment medium.

The body 11 has a first and a second face 12, 14 and comprises a plurality of channels extending in a direction from the first face 12 to the second face 14. The faces 12, 14 are of a flat design and arranged essentially in parallel.

The plurality of channels comprises a number of channels 16 of a first type and a number of channels 18 of a second type.

The channels 16 of the first type are open at one end located at the first face 12 and closed at the other end located adjacent to the second face 14.

The channels 18 of the second type are open at one end located at the second face 14 whereas the other end of the second type of channels 18 is closed adjacent to the first face 12 of the body 11.

Fluid to be treated will enter the channels 16 permeate through the bulk of the body 11 of fluid pervious treatment medium and exit the body 11 through the second type of channels 18 at the second face 14 of the body 11. In contrast to conventional fluid treatment elements the fluid flow through the fluid treatment medium does not occur in a perpendicular direction to its surface but in a substantially parallel or edgewise direction.

The body 11 of fluid treatment medium may be sealed at its out peripheral surface 20. Alternatively, the filter element may be incorporated into the fluid treatment module with the outer periphery in a compressed state, such that fluid to be treated will rather flow in the direction to the channels 18 than in the direction to the peripheral surface 20.

The channels in the body 11 may have a simple circular cross section but any other cross section, e.g., oval or polygonal may also be suitable.

Channels of a circular or oval cross section are more easily provided in the body 10 by drilling whereas more complex geometries of the channel cross sections may be achieved by, e.g., laser etching.

The channels 16 are shown with an about 2.5 times larger diameter as compared to the diameter of the channels 18. A design where the channels 16 receiving the non-treated fluid have a substantial larger diameter than the channels 18 receiving the treated fluid has the advantage that they may accommodate a substantial amount of deposits prior to clogging.

In such a case it is further more preferable to provide a number of channels 18 which is larger than the number of channels 16 as shown in Figure 1.

The fluid pervious treatment medium may be selected from a broad variety of media, e.g., meltblown media (like media based on polypropylene which are used for PALL HDC cartridges), other wet-laid and dry-laid media.

Specific examples of depth filter media suitable for use in the present invention are cellulose based depth filter media of the types K700 and T1500 which are available from Pall Corp.

The fluid treatment medium will in many cases be of the depth filter type which may be used in a broad range of treatment applications including but not limited to depth filtration. Additional applications especially include adsorptive separation, chromatographic separation, surface (sieving) filtration or any combinations thereof.

While the fluid treatment element 10 shown in Figure 1 is of a unitary structure, Figure 2 shows an embodiment of the present invention where the fluid treatment element 30 comprised three distinct components.

The fluid treatment element 30 comprises a first type of layer 32 and a second type of layer 36 and a body 34 of fluid pervious treatment medium which is located in between the first and second type of layers 32, 36. The first and second type of layers 32, 36 are located at the first and second faces 38, 40 of the body 34, respectively.

The stack of the layer 32, the body 34 and the layer 36 is shown in Figure 2 in an exploded view.

Instead of a single layer 32, the layer 32 may be provided as multilayer structure and the same is true for the layer 36 which may also be replaced by a multilayer structure. When the layers 32 and/or 36 have a multilayer structure, not all of the layers of the multilayers need to be of the same type. Different layers may be used to further adapt the fluid treatment element to the specific demands of an application.

The same is true for the selection of the material to constitute the first and second layers 32, 36.

The layers 32 and/or 36 may be pervious or impervious to the fluid to be treated. In case the layers 32, 36 are selected from fluid pervious material they are designed to provide a substantially similar treatment to the fluid passing through the bulk of these layers from the openings 42/channels 38 to the channels 40/openings 50.

The body 34 comprises parallel channels 42 and 44, the channels 42 receiving the non-treated fluid whereas the channels 44 receive the fluid after it has passed through the body 34 of fluid pervious medium 46.

Both types of channels 42 and 44 extend from the first face 38 to the opposite second face 40 of the body 34 and will be closed at one end by portions of the first layer 32 and the second layer 36 adjacent to the first face 38 and the second face 40, respectively.

Layer 32 has openings or through holes 50 which are oriented in an assembled state of the fluid treatment element 30 such that they are in line with the ends of the channels 42 of the body 34 at the first face 38.

The other ends of the channels 42 of the body 34 adjacent to the second face 40 and facing the second layer 36 are closed by the medium of this layer 36.

At the opposite face of the body 34 the second type of layer 36 is mounted such that openings 52 provided in layer 36 are in line with the ends of channels 44 at the second face 40 of the body 34. Thereby, drainage of the channels 44 of the body 34 is provided while those portions of the second face 40 of the body 34 incorporating the ends of the channels 42 are closed by portions of the layer 36. Again in an assembled state the fluid treatment element 30 requires that the fluid to be treated enters the openings 50 and flows into the channels 42, then passes through the fluid pervious treatment medium 46 in an edgewise direction and is subsequently collected in channels 44. From the channels 44 the treated fluid is discharged through the openings 52 of layer 36.

The same comments as to the selection of the dimensions of the channels 42, 44 apply as has been set out in connection with the embodiment of Figure 1.

The basic concept of a fluid treatment element 30 of Figure 2 has the advantage over the embodiment of Figure 1 that by providing not only one body 34 in between the layers 32 and 36 but two or more the treatment capacity of the element 30 may be easily varied and adapted to the needs of a specific application.

The outer periphery 54 of the fluid treatment element 30 may be sealed in order to prevent fluid to exit the fluid treatment element 30 other than through the channels 44 and openings 52. This may be effected by applying a sealing layer at the outer periphery 54 or by compressing the stack of layers 32, 36 and the body 34 to a sufficient extent.

The fluid treatment elements 10 and 30 may be provided with a fluid inlet and a fluid outlet, e.g., by incorporating the same into a housing to form an inventive fluid treatment module, said housing incorporating such fluid inlet and fluid outlet.

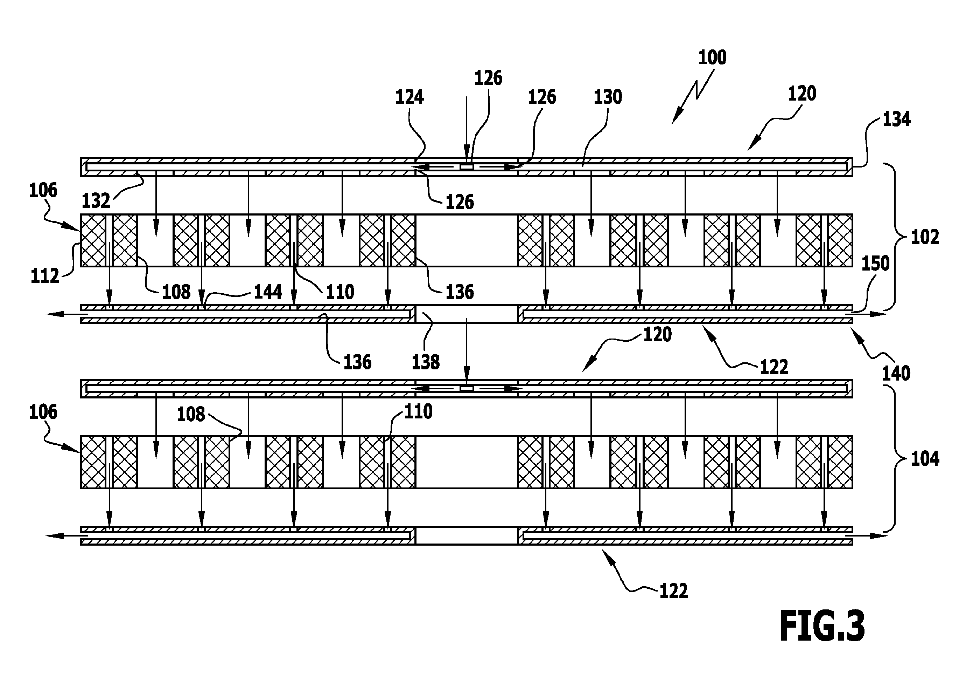

Figure 3 shows a schematic representation of a fluid treatment assembly 100 according to the present invention in an exploded view incorporating two fluid treatment elements 102 and 104. The fluid treatment modules 102 and 104 are of the same structure and may be stacked on top of one another in order to duplicate the treatment capacity of the fluid treatment assembly 100. It is well apparent from Figure 3 that the number of fluid treatment modules still may be increased and thereby the treatment capacity of the fluid treatment assembly may be adapted to the needs of a specific application.

Each of the treatment modules 102 and 104 is comprised of a body 106 of a fluid pervious treatment medium 112 comprising a plurality of channels of two types 108 and 110 transecting the fluid pervious treatment medium of the bodies 106. The bodies 106 are sandwiched in a stack in between two end pieces 120, 122, the first end piece 120 being located at the upstream face of the body 106 whereas the end piece 122 is located at the opposite face of the body 106.

The end piece 120 has a plate shaped hollow structure extending essentially over all of the area of the body 106 at its upstream side or first face.

The end piece 120 has a central opening 124 with a cylindrical wall including one or more perforations 126 providing a fluid pathway for fluid to be treated from the opening 124 into an interior space 130 of the hollow end piece 120. The interior space 130 is essentially coextensive with the extension of the end piece 120 and the fluid treatment element 106. At the outer periphery 134 the end piece 120 and its interior space 130 are closed.

While the end piece 120 is of a closed structure at one face (in Figure 3 the upper face), on the opposite face of the end piece 120 contacting the body 106 in the assembled state openings 132 are provided through which fluid to be treated may exit the interior space 130 of the end piece 120 and enter corresponding channels 108 receiving the non-treated fluid. Thus, the end piece 120 provides in addition to a fluid inlet for the fluid treatment modules 102, 106 the functionality of a first layer 32 of the fluid treatment element 30 described in connection with Figure 2.

The body 106 is provided with a central opening 136 which may be provided with a closed or fluid impervious cylindrical coating or wall restricting the fluid flow into the body 106 and to the pathways provided by the channels 108. Alternatively, in case the body is made from a compressible medium the body may be compressed to an extent that the fluid flow is limited in the same way.

Likewise the body 106 may be provided at its outer periphery with a fluid impervious coating or wall or may be compressed (where possible) to an extent that the fluid flow within the body is restricted to fluid flow paths from the channels 108 to the channels 110.

The end piece 122 is also of a plate shaped hollow structure and includes a central opening 138 the circular wall of which is of a closed or fluid impervious structure.

At the outer periphery 140 of the end piece 122 one or more openings 150 are provided in regular intervals serving for draining the interior space 136 of the end piece 122.

On one of the end faces of the end piece 122 there is a closed, fluid imperious wall extending over the whole dimension of the end piece 122 from the central opening 138 to the outer circumference 140.

On the other end face of the end piece 122 openings 144 are provided which in the assembled state of the module 102 and 104 will meet with one end of the channels 110 to receive the treated fluid exiting from the body 106.

The treated fluid enters the end piece 122 through the openings 144 and flows in a radial direction outwardly to the circumference 140 of the end piece 122 where it exits through one or more openings 150.

The central openings 124, 136 and 138 of the first end piece 120, the body 106 and the second end piece 122, respectively, are designed to match with their cross sections so that when the module 102 is assembled an essentially straight channel is provided by these openings which allows to feed non-treated fluid into the stack of modules 102, 104 and optionally further fluid treatment modules. The cross section of these openings 124, 136, 138 is designed such that a sufficient fluid flow may be provided to sufficiently supply all of the fluid treatment modules in a common stacked configuration.

In an alternative embodiment the fluid treatment modules 102, 104 may have in addition to the end pieces 120, 122 first and second layers as shown in and described in connection with the embodiment of a fluid treatment element 30 of Figure 2. Of course, said layers would have to be provided with central openings designed to match the openings 124 and 138 of the end pieces 120, 122 and the opening 136 of the body 106.

Of course the fluid flow may be directed in just the opposite way, i.e., the end pieces with the one or more openings 150 at the outer periphery 140 could receive the non-treated fluid and distribute it into channels 108 receiving the non-treated fluid. Of course the openings 144 would be arranged and designed to match with the channels 108. The design of the other end piece 120 would have to be adapted in a corresponding manner.

Fluid flow through the body 106 then would be essentially the same whereas the end piece 120 would receive the treated fluid and discharge the same through the central opening 124.

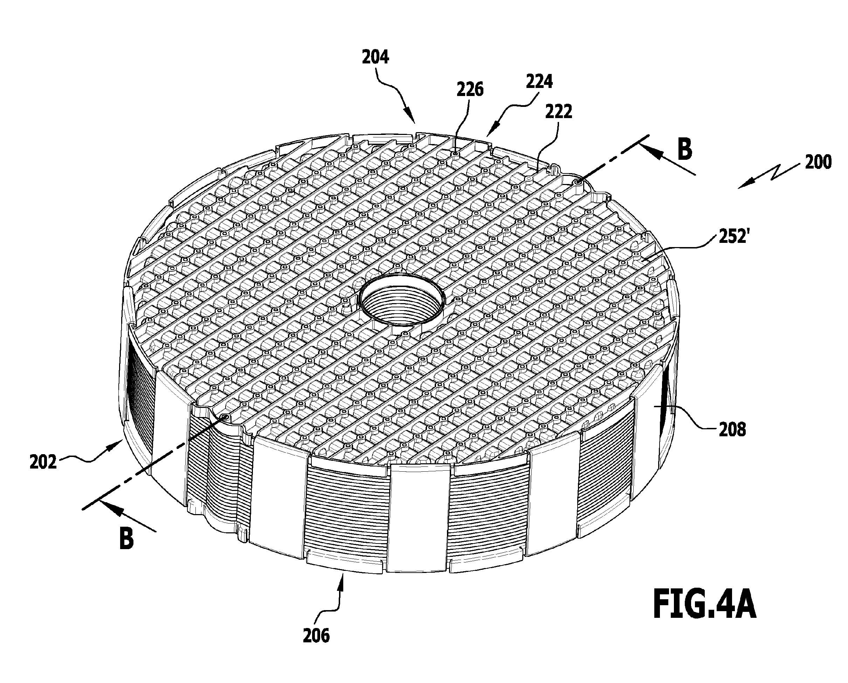

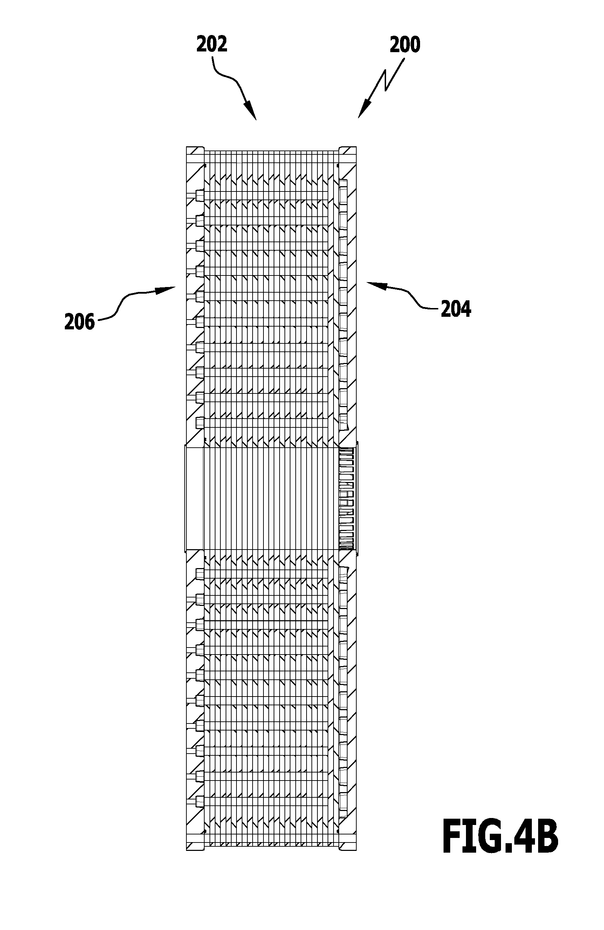

Figures 4 to 7 relate to an inventive fluid treatment module 200 comprising a fluid treatment element 202 comprising a stack of a multiplicity of disc shaped layers of a fluid treatment medium. The number of layers may amount to, e.g., 24 layers.

At the top and bottom of the fluid treatment element 202 disc shaped end pieces 204, 206 are provided which are connected by clamping elements 208 regularly distributed along the periphery of the fluid treatment module 200. The clamping elements 208 are designed to keep the module 200 and the stack of disc shaped layers of the fluid treatment element 202 in a compressed state.

Figure 4A shows the module 200 in a perspective representation, while Figure 4B shows a cross-section of the module 200 along line B - B in Figure 4A.

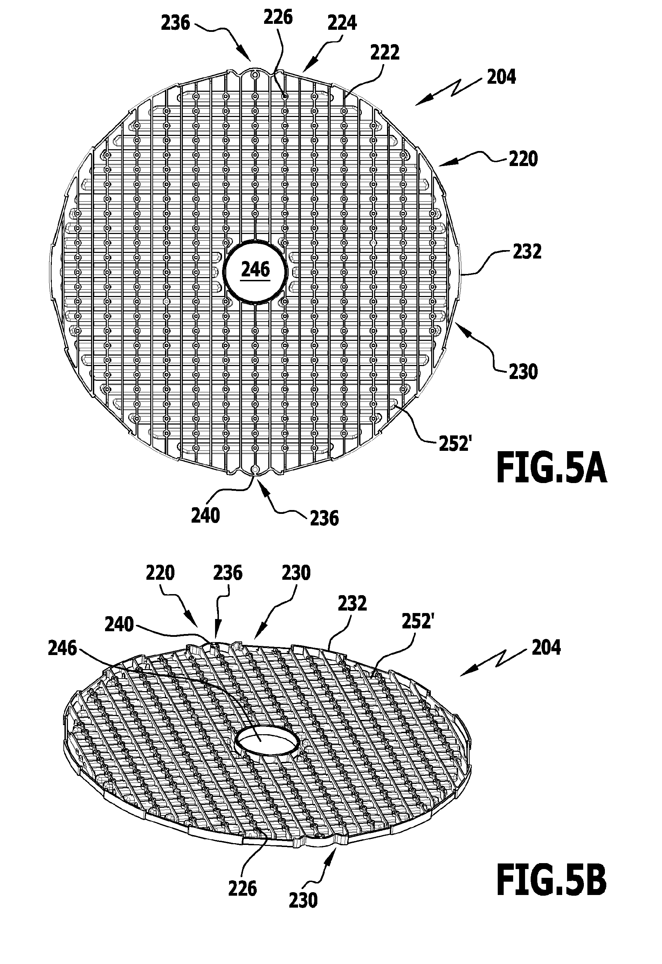

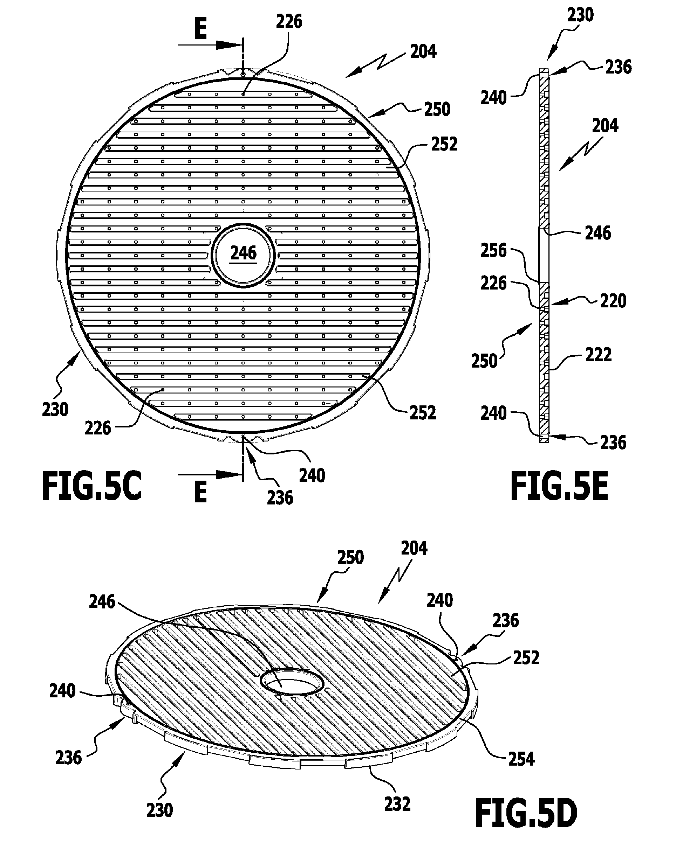

The various components of the module 200 and the fluid treatment element 202 will be discussed with reference to Figures 5 to 7 in more detail.

Figures 5A and 5B show the outer face 220 of the upper end piece 204 of the module 200 in a top view and a perspective representation, respectively.

As is apparent from Figures 5A and 5B (as well as from Figure 4A) the outer face 220 of the upper end piece 204 is provided with a plurality of parallel ribs 222 extending across the whole surface of the end piece 204. The ribs 222 provide for a mechanical stability of the upper end piece 204 such that it may substantially maintain its plane configuration when the module 200 is hold in its compressed state by the clamping elements 208. Thereby the compressional forces acting on the various portions of the fluid treatment element 202, especially also adjacent to the outer and inner periphery of the fluid treatment module 200 are substantially the same.

Between the ribs 222 ducts 224 are provided which extend in parallel to the ribs 222 substantially across the whole outer face 220 of the end piece 204. In the middle of the ducts 224 through holes 226 are provided in a parallel row serving to feed non-treated fluid to the top face of the fluid treatment element 202.

The upper end piece 204 comprises at the outer periphery 230 a plurality of projections 232 which receive one end of the clamping elements 208 in a form-fit configuration.

The central portion of the upper end piece 204 comprises a central passage 246 with a circular cross-section.

At two opposite parts of the outer periphery of the upper end piece 204 radially extending projections 236 (optionally) are provided which serve in cooperation with corresponding projections of the lower end piece 206 as well as the various components of the fluid treatment element 202 as a means to easily compose the module of the various parts with a precise orientation. For ease of use the projections 236 may be provided with bore or gap 240 which may accommodate a bolt-like element during assembly of the module (not shown).

The upper end piece 204 with its inner face 250 is shown in Figures 5C and 5D. Figure 5E provides a cross-sectional view along line E - E in Figure 5C.

At the inner face 250 a plurality of parallel ducts 252 is provided which extend substantially across the whole area of the inner face 250. These ducts can be seen as ribs 252' at the outer face 220 of the end piece 204. The ducts 252 are arranged in direction perpendicular to the ribs 222 and ducts 224 on the outer face 220 of the upper end piece 204.

The outer periphery 230 of the end piece 204 may have a circular rim 254 projecting perpendicular from the inner face 250 serving to provide a somewhat higher compression of the layers of the fluid treatment element 202 at the outer periphery thereof.

Similarly, the central passage 246 may be provided with a circular rim 256 projecting perpendicular form the inner face 250 which likewise serves to provide a somewhat higher compression of the layers of the fluid treatment element 202 at their central portion.

The provision of the circular rims 254, 256 may in some embodiments be sufficient to seal the outer and inner surface of the fluid treatment element so that not sealing layers of wall portions are needed to ensure an exclusive fluid flow within the body of layers of the fluid treatment element 202.

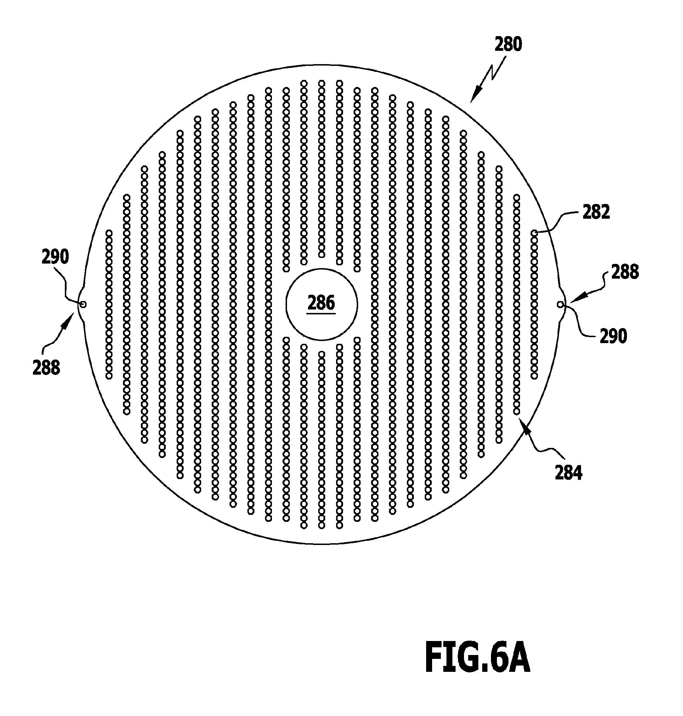

Figures 6A to 6C show three different types of layers 280, 300 and 320 which constitute the stack of layers of the fluid treatment element 202.

One or more of the layer 280, e.g., three layers 280, may be arranged in the stack of layers at the upstream end of the fluid treatment element 202, i.e., adjacent to the upper end piece 204.

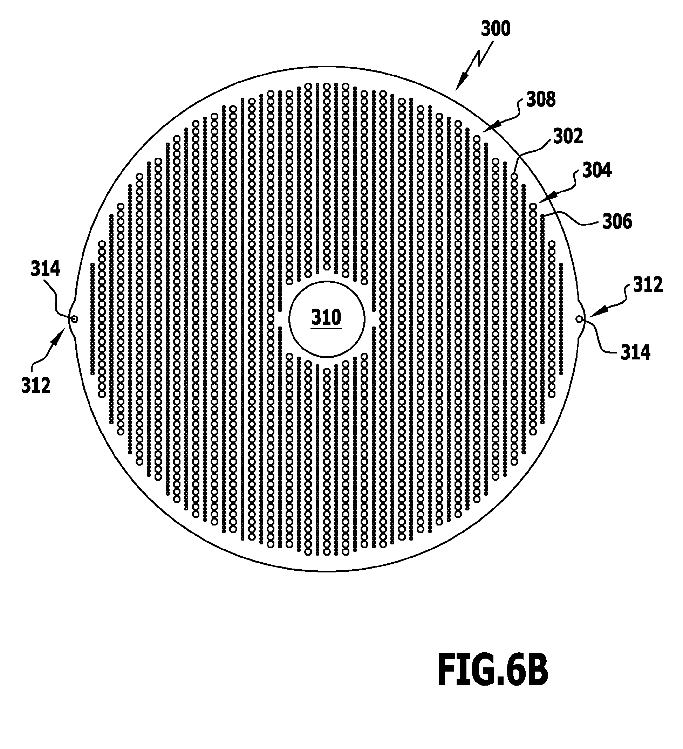

The vast majority of layers of the stack of the fluid treatment element 202 is composed of layers 300 (Figure 6B) which form the body of the fluid treatment element 202. For example, the fluid treatment element 202 may comprise about twenty layers 300.

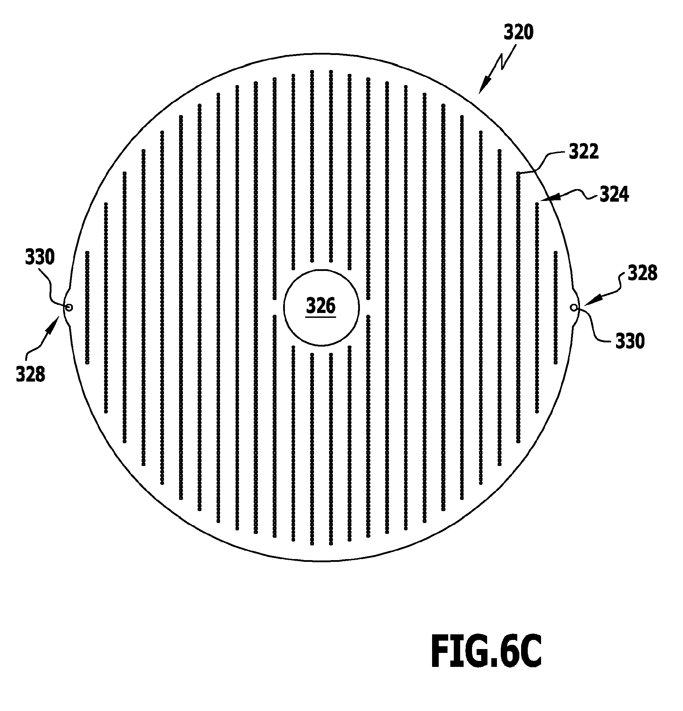

At the downstream end of the fluid treatment element 202 one or more layers 320 (cf. Figure 6C), e.g., three layers 320, may be arranged in the stack adjacent to the lower end piece 206 of the fluid treatment module 200.

The layer(s) 280 comprise a plurality of large openings 282 arranged in parallel rows 284. The diameter of the openings 282 may be, e.g., about 5 mm. The distance of the centers of two openings of such dimensions may be, e.g., about 6 mm.

The layer 300 comprises the same plurality of large openings 302, arranged in parallel rows 304 in a substantially identical pattern as the openings 282 of the layer(s) 280. The openings 302 may have the same diameter as the openings 282 of the layer(s) 280.

The layer 300 furthermore comprises small openings 306 arranged in parallel rows 308. The rows 308 extend in parallel to and are positioned in between the rows 304 of large openings 302. The small openings may have a diameter of, e.g., about 2 mm. The distance of the centers of two openings of such dimensions may be, e.g., about 3 mm.

As is apparent from Figure 6B the number of the small openings 306 is about double the number of the large openings 302.

The layer 320 of which one or more, e.g., three layers 320, may be provided in the stack of the fluid treatment element 202 comprises small openings 322 in parallel rows 324 in a pattern matching with the pattern of the small openings 306 of layer 300.

All of the layers 280, 300 and 320 are provided with a central passage 286, 310 and 326, respectively, all preferably matching in position and size with the central passage 246 of the upper end piece 204.

Furthermore, all of the layers 280, 300, 320 are optionally provided with two projections 288, 312, 328 radially extending from the layers 280, 300, 320 in opposite directions. The projections serve to facilitate assembly of the stack of layers 280, 300, 320 and positioning them in a precise alignment with the upper and lower end pieces 204, 206. More preferably, the projections 288, 312, 328 are provided with a bore or gap 290, 314, 330, respectively, designed to receive a bolt-like element during assembly.

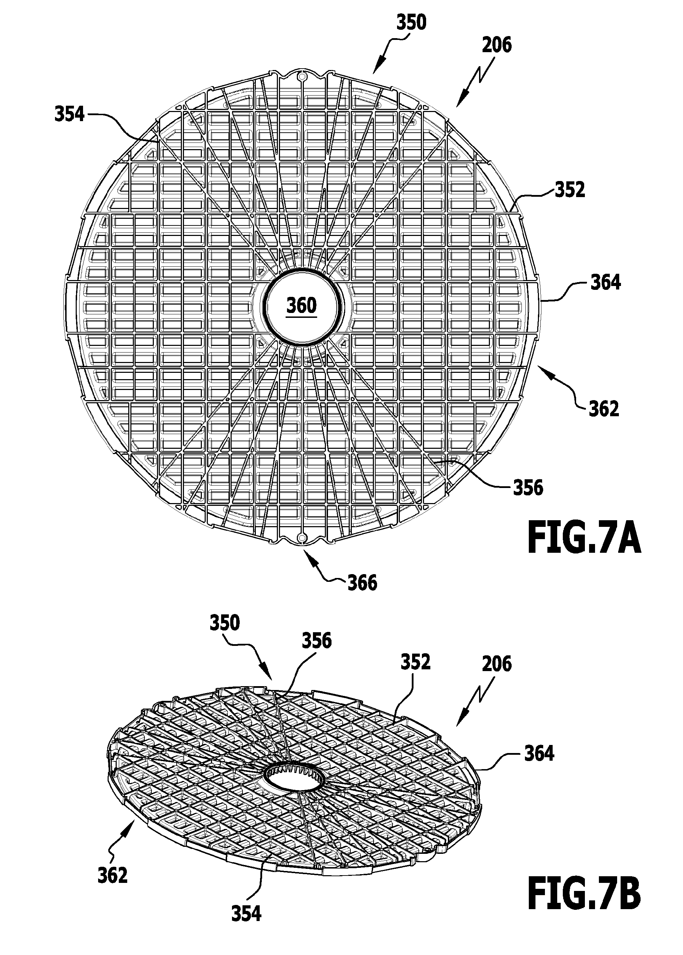

Figures 7A to 7F show the lower end piece 206 in several representations. Figure 7A provides a top view and Figure 7B provides a perspective view of the outer (bottom) face 350 of the lower end piece 206.

The outer face 350 comprises a plurality of ribs extending all across the area of the face 350, one group of ribs 352 being arranged in parallel and regularly spaced from one another, whereas a second group of ribs 354 arranged in parallel and regularly spaced from one another extend in perpendicular direction to the direction of the first group of ribs 352 likewise all across the area of the face 350.

A third group of ribs 356 extends substantially in a radial direction from a central passage 360 of the end piece 206 to the outer periphery 362 thereof.

At the outer periphery 362 the end piece 206 comprises a number of projections 364 extending perpendicular from the outer face 350. These projections 364 are designed to receive one end of the clamping elements 208 in a form-fit configuration.

At two opposite sections of the outer periphery 362 the end piece 206 is optionally provided with projections 366 serving upon assembly of the various components of the fluid treatment module 200 to correctly align the other components with the lower end piece 206. Preferably, the projections 366 are provided with a bore or gap 368 to receive a bolt-like element (not shown) during assembly.

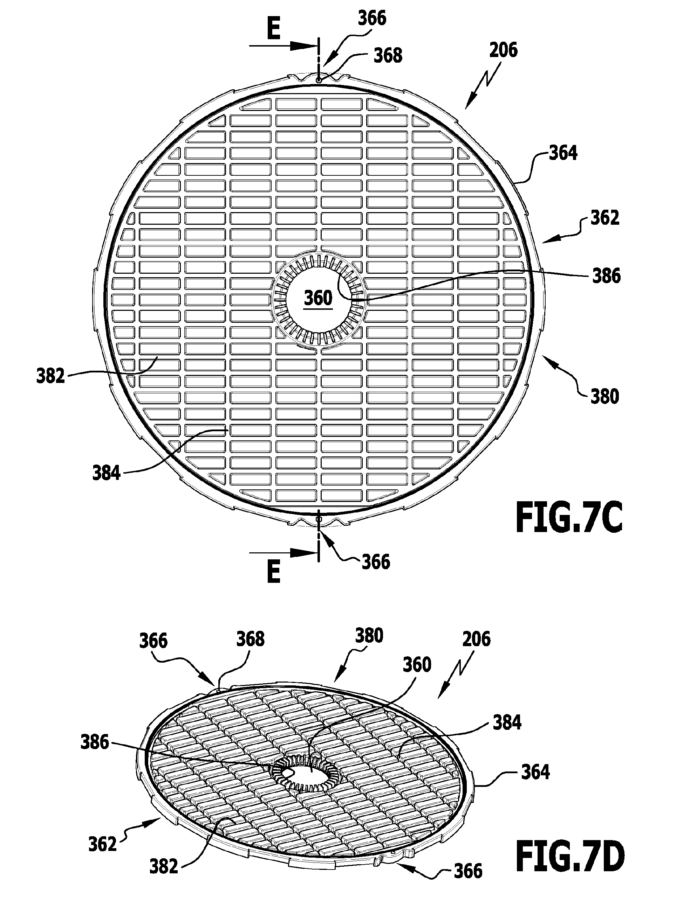

The lower end piece 206 is structured on its inner face 380 (Figures 7C and 7D) so as to provide a fluid receiving space receiving the treated fluid from the fluid treatment element 202.

At the same time the lower end piece 206 has to support the fluid treatment element 202 and its body of fluid treatment material.

In order to cope with these requirements, the lower end piece 206 is provided with a network of two types of grooves 382 and 384 extending essentially across the whole area of the lower end piece 206 and arranged perpendicularly to one another.

Such structure in addition increases the stiffness of the lower end piece 206.

Adjacent to the central passage 360, a plurality of radially oriented ribs 386 are provided which support the body of treatment medium of the fluid treatment element 202 at its innermost area and at the same time provide a fluid communication for the network of grooves 382, 384 to the central passage 360.

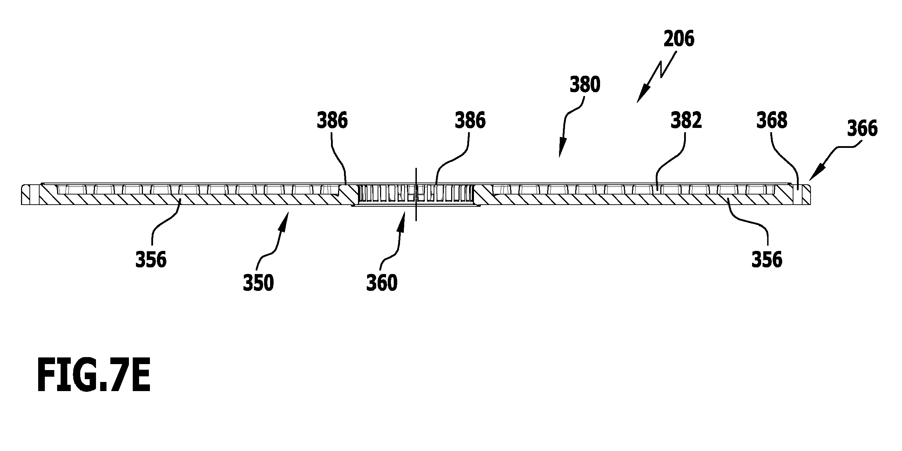

Figure 7E shows a cross-section of the lower end piece 206 along the line E - E in Figure 7C.

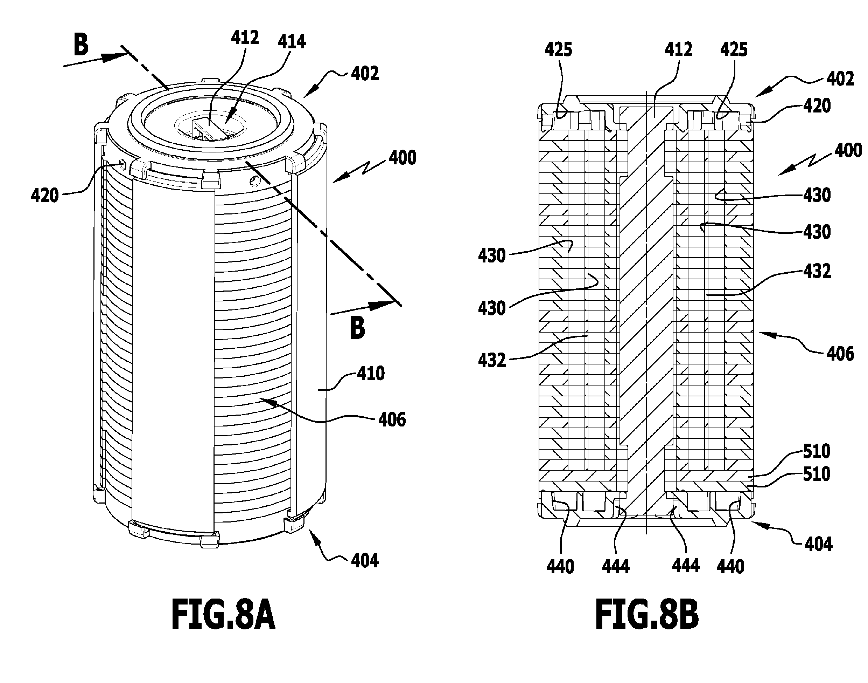

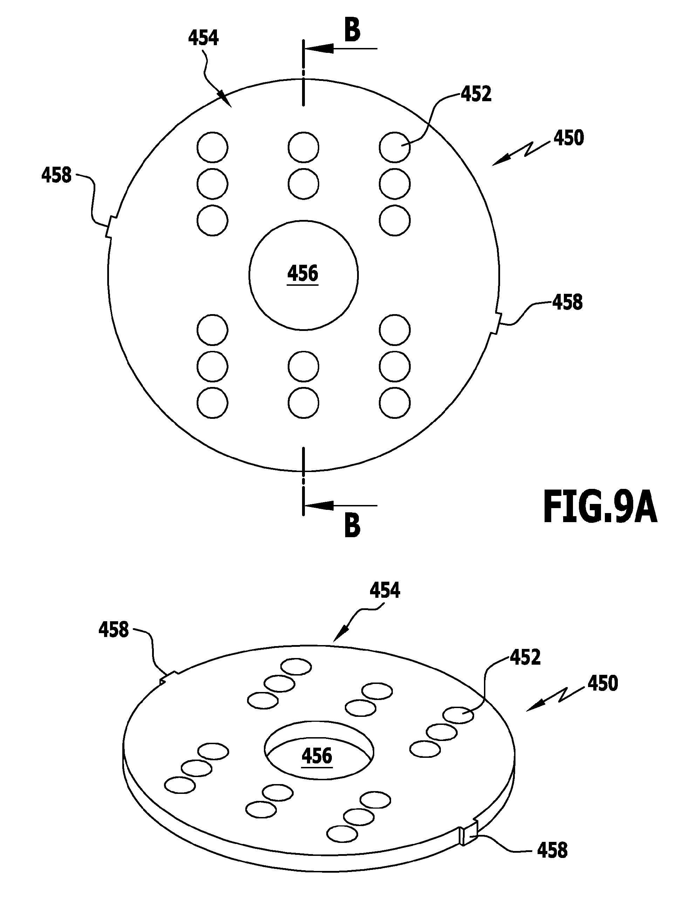

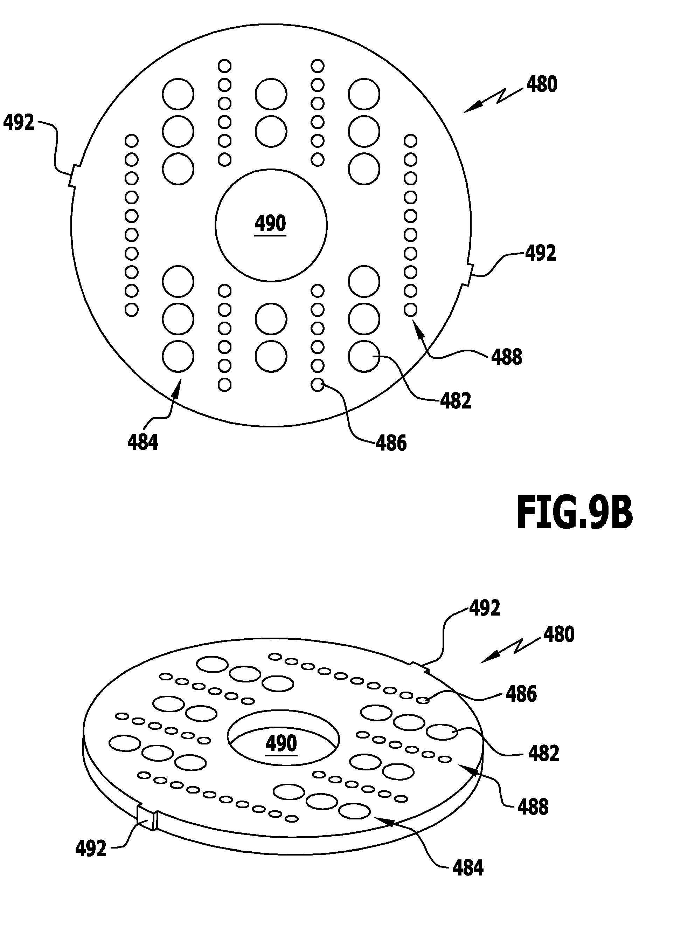

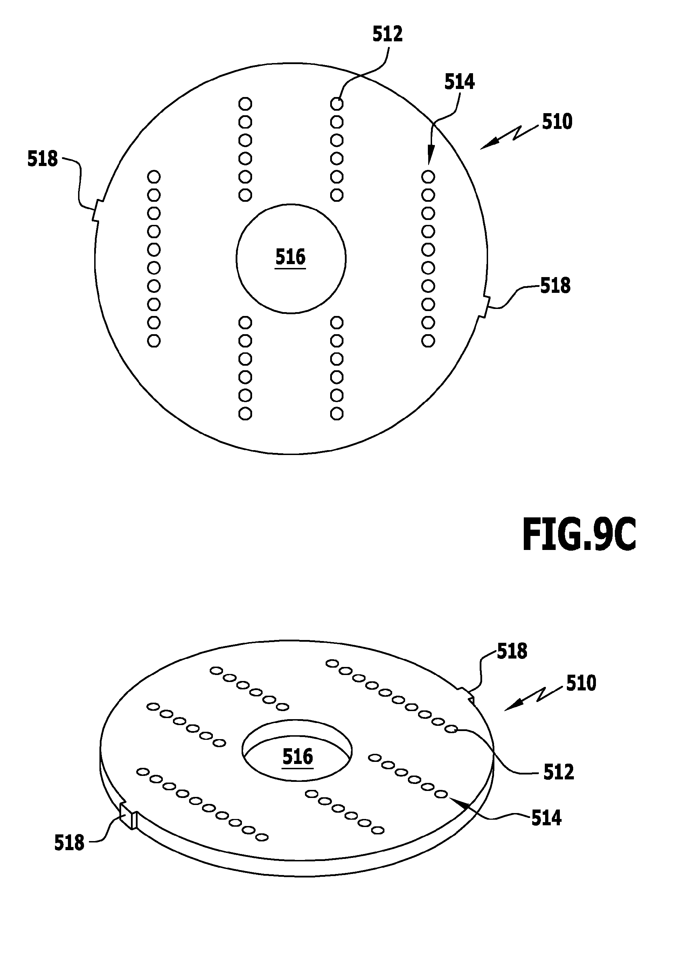

Figures 8 and 9 show a further specific embodiment of an inventive fluid treatment module 400. The treatment module 400 typically will be provided in a smaller size than the module 200 and serves, e.g., for laboratory purposes, while the larger size of the module 200 may be adapted to sizes fit for industrial scale treatments.

The fluid treatment module 400 comprises a first and a second end cap 402, 404 and a fluid treatment element 406 of a multilayer structure arranged in between the end caps 402, 404.

The individual parts 402, 404, 406 of the module 400 are secured in their mounted positions by clamping elements 410, which may be connected to the end caps 402, 404 in a form fit arrangement.

The structure of the end caps 402 and 404 is more simple than the structure of the end pieces of the module 200. In order to secure a fluid tight contact between the end caps 402, 404 and the fluid treatment element 406 as well as within the multilayer structure of the fluid treatment element 406 an additional central clamping element 412 may be used which extends through a central duct 414 of the module 400 from one end cap to the other.

The end cap 402 receives non-treated fluid via the openings 420 which are provided at the outer periphery of the end cap 402 in a regular arrangement.

The fluid inlet openings 420 lead into the interior of the end cap 402 which provides a fluid distribution space 425 which is in fluid communication with a plurality of channels 430 (feed channels) extending in the direction from one end cap 402 to the other end cap 404. The channels 430 are separated from one another by a stay 432 of fluid treatment medium of the layers of the filter treatment element 406. Channels receiving the treated fluid (treated fluid collecting channels) are not visible in this cross-sectional representation but are arranged in parallel to the channels 430.

The channels 430 extend over essentially the whole axial length of the module 400 and are closed at one end by two layers 510 of a treatment medium which are shown in more detail in the Figure 9C. These layers 510 do have (not apparent from Figure 8B but clearly shown in Figure 9C) openings forming part of the channels receiving the treated fluid and providing a fluid communication to a treated fluid collecting space 440 provided within the lower end cap 404.

The treated fluid collecting space 440 may be drained through one or more fluid outlets 444 into a central passage of the lower end cap 404.

Figures 9A to 9C, similarly to Figures 6A to 6C show three different types of layers 450, 480, and 510 which constitute the stack of layers of the fluid treatment element 406. The number of layers in the stack may be varied in a broad range, e.g., of from 6 up to 34.

One or more of the layer 450, e.g., two layers 450, may be arranged in the stack of layers at the upstream end of the fluid treatment element 406, e.g., adjacent to the upper end cap 402.

The vast majority of layers of the stack of the fluid treatment element 406 is composed of layers 480 (Figure 9B) which form the body of the fluid treatment element 406. For example, the fluid treatment element 406 may comprise about thirty layers 480.

At the downstream end of the fluid treatment element 406 one or more layers 510, e.g., two layers 510, may be arranged in the stack adjacent to the lower end cap 404 of the fluid treatment module 400.

The layer(s) 450 comprise a plurality of large openings 452 arranged in parallel rows 454. The diameter of the openings 452 may be, e.g., about 5 mm. The distance two adjacent openings of such dimensions may be, e.g., about 1 mm.

The layer 480 comprises the same plurality of large openings 482, arranged in parallel rows 484 in a substantially identical pattern as the openings 452 of the layer(s) 450. The openings 482 may have the same diameter as the openings 452 of the layer(s) 450 and are arranged at the same distance.

The layer 480 furthermore comprises small openings 486 arranged in parallel rows 488. The rows 488 extend in parallel to and are positioned in between the rows 484 of large openings 482. The small openings 486 may have a diameter of, e.g., about 2 mm. This distance two adjacent openings of such dimensions may be, e.g., about 1 mm.

As is apparent from Figure 9B the number of the small openings 306 is about double the number of the large openings 482. The number of rows is larger than the number of rows 484.

The layer 510 of which one or more, e.g., two layers 510, may be provided in the stack of the fluid treatment element 406 comprises small openings 512 in parallel rows 514 in a pattern matching with the pattern of the small openings 486 of layer 480.

All of the layers 450, 480 and 510 are provided with a central passage 456, 490, 516, respectively, all preferably matching in position and size with the central passages of the upper and lower end caps 402, 404.

Furthermore, all of the layers 450, 480, 510 are optionally provided with two projections 458, 492, 518, respectively, radially extending from the layers 450, 480, 510, respectively, in opposite directions. The projections serve to facilitate assembly of the stack of layers 450, 480, 510 and positioning them in a precise alignment with the upper and lower end pieces 204, 206.

The fluid treatment modules 200 and 400 according the embodiments of Figures 4 to 7 and 8 and 9, respectively, provide a significantly improved treatment capacity, e.g., a filtration capacity which is about double as compared to the filtration capacity of a filter module having the same dimensions and where the fluid flow is directed perpendicular to the surfaces of the layers of the treatment medium.

Most importantly, the fluid treatment modules of the present invention provide a significant volume to accommodate sediments of heavily loaded fluids to be treated and thereby unexpectedly improve the fluid treatment/filtration capacity and characteristic of the modules.

The provision of the feed channels and treated fluid receiving channels extending through the bulk of the fluid treatment medium and thereby providing a fluid flow edgewise to the layer structure provides a further significant advantage over the conventional filter modules.

## Claims

1. A fluid treatment module comprising a fluid treatment element, a fluid inlet for non-treated fluid and a fluid outlet for treated fluid,
said fluid treatment element comprising a body of a fluid pervious treatment medium with a first and a second face said first and second face being positioned at opposite sides of the body,
said body comprising a plurality of channels extending in a direction from the first face to the second face,
wherein a number of channels of a first type of said plurality of channels is open at one end located at said first face of the body and closed at the other end located adjacent to second face,
wherein a number of channels of a second type of said plurality of channels is open at one end located at said second face of the body, and closed at the other end located adjacent to said first face,
wherein said channels of the first type are separated from the channels of the second type by portions of the fluid pervious treatment medium,
one of said types of channels being feed channels receiving non-treated fluid and communicating with the fluid inlet of said fluid treatment module, the other one of said types of channels being outlet channels collecting treated fluid and communicating with said fluid outlet of said fluid treatment module.

2. The fluid treatment module of claim 1, wherein said body comprises a plurality of flat stacked layers of a sheet material, said layers comprising openings forming said plurality of channels.

3. The fluid treatment module of claim 1 or 2, wherein said fluid treatment element comprises one or more layers of a first type of layers located at and positioned in parallel to the first face of the body, said first type of layers having openings corresponding only to one type of channels and/or said fluid treatment element comprises one or more layers of a second type of layers located at and positioned in parallel to the second face of the body, said second type of layers having openings corresponding only to the other type of channels, optionally
wherein said first and second type of layers are made of a fluid treatment medium the composition and/or structure of which is preferably similar to or identical with the treatment medium of the body of the treatment element.

4. The fluid treatment module of any one of claims 1 to 3, wherein the cross sections of each type of channels are substantially of the same size.

5. The fluid treatment module of any one of claims 1 to 3, wherein the cross sections of the channels communicating with the fluid inlet are of a larger size than the cross sections of the channels communicating with the fluid outlet, preferably
wherein the difference in size of the cross sections amounts to about 10 % or more, based on the size of the cross section of the channels communicating with the fluid outlet.

6. The fluid treatment module of any one of claims 1 to 5, wherein the number of channels communicating with the fluid inlet is smaller than the number channels communicating with the fluid outlet, preferably
wherein the number of channels communicating with the fluid outlet is about twofold or more as compared to the number of channels communicating with the fluid inlet.

7. The fluid treatment module of any one of claims 1 to 6, wherein the channels in the fluid treatment medium are arranged in separate parallel or concentric rows for each type of channels, optionally
wherein the channels communicating with the fluid outlet are arranged in groups of two or more adjacent rows, whereas the channels communicating with the fluid inlet are arranged in groups of a lesser number of rows.

8. The fluid treatment module of any one of claims 1 to 7, wherein the fluid treatment medium is a depth filter material which is preferably maintained in a compressed state, preferably
wherein the compression of the fluid treatment medium is such that the thickness of the body of treatment medium amounts to about 90 % or less of the thickness of the non-compressed depth filter material.

9. The fluid treatment module of any one of claims 1 to 8, wherein the fluid treatment medium comprises a matrix including a compressible material and/or a material which swells in contact with the fluid to be treated.

10. The fluid treatment module of any one of claims 1 to 9, wherein the fluid treatment medium comprises a matrix incorporating an additive, said additive being preferably in particulate or fibrous form, preferably wherein the additive is present in an amount of about 15 to about 70 % by weight, based on the weight of the fluid treatment medium, optionally
wherein the additive is selected from a porous particulate additive, a filter aid, and/or a treatment agent and/or
wherein the additive comprises a reactive agent, an adsorptive agent, preferably
wherein the additive is selected from kieselguhr, perlite, fine cellulose, micro-crystalline cellulose, nanofibers, activated carbon, adsorber beads, fibrets and fibrids.

11. The fluid treatment module of any one of claims 1 to 10, wherein the fluid treatment medium comprises a matrix said matrix including organic polymer material.

12. The fluid treatment module of any one of claims 1 to 11, wherein the module comprises two end pieces to be sealingly positioned with said first and second faces, said end pieces providing the fluid inlet and fluid outlet, respectively.

13. The fluid treatment module of any one of claims 1 to 12, wherein said module comprises clamping means positioned on the outer peripheral surface of the module, said clamping means preferably maintaining the fluid treatment material of the module in a compressed state, optionally wherein said clamping means comprise a clamping element positioned in a central duct of the module.

14. A fluid treatment assembly comprising two or more fluid treatment modules of any one of claims 1 to 13, preferably said fluid treatment modules being arranged in a stack, optionally
wherein said modules comprise a common central conduit in fluid communication with the fluid inlets or fluid outlets of any one of the fluid treatment modules of the system.

15. The fluid treatment assembly of claim 14, wherein said assembly comprises a retention device, preferably positioned on the outer peripheral surface of the stack of modules, said retention device preferably maintaining the fluid treatment material of the stack of modules in a compressed state, optionally wherein said assembly comprises a retention element positioned in said central conduit provided by the central ducts of the modules.

16. A process for treating a fluid, said method comprising arranging a fluid treatment module of any one of the claims 1 to 13 or a fluid treatment assembly according to claim 14 or 15 such that the fluid inlets of the modules are on top and said channels of the fluid treatment element(s) of the module(s) being oriented in an about vertical direction, and feeding the fluid to be treated into the fluid inlet of the module and the assembly, respectively.

17. The process of claim 16, wherein the channels of the module(s) in fluid communication with the fluid inlet capture and accommodate sediment from the fluid to be treated.
